# EUROPEAN PATENT APPLICATION

(11) **EP 3 521 413 A1**
(43) Date of publication of application: **07.08.2019**
(21) Application number: 16917189.9
(22) Date of filing: 29.09.2016
(51) Int. Cl.: C12M 1/33, C12M 3/08

(54) **DEVICE FOR ISOLATING CELLS FROM TISSUE**

(71) Applicant: Kaohsiung Medical University, Kaohsiung 807 (TW)
(72) Inventor: LIN, Che-hsin, Kaohsiung 813 (TW); WANG, Yao-hsien, Kaohsiung 825 (TW); CHEN, Chung-hwan, Kaohsiung 801 (TW); CHANG, Je-ken, Kaohsiung 807 (TW); HO, Mei-ling, Kaohsiung 813 (TW)
(74) Representative: Lang, Christian
(86) International application number: PCT/CN2016/100816
(87) International publication number: WO 2018/058435

(57) **Abstract**

Provided is a device for rapidly isolating cells from tissue, comprising: a stirring and cutting member, having a rotary cutting tool, wherein same is driven by a motor so as to rotate to stir and cut tissue, and can inject digestive enzymes to accelerate decomposition of the tissue; and double-layered nested cups comprising an inner cup and an outer cup, used for accommodating and filtering solution of required cells, wherein a filter device of the inner cup screens out the required cells, and the outer cup collects the filtered solution.

## Description

### Technical Field

The present invention relates to a device capable of rapidly separating cells from tissue, and in particular to a device capable of separating cells or stem cells from animal tissues or fats

### Background

In the prior art for separating cells from tissues, it is often used to manually cut the tissue by using scissors followed by decomposing the tissue through an enzyme reaction, so that the cells can be released from the tissue, and then centrifugal separation is carried out to obtain the released cells. The method is quite time-consuming and labour-consuming, wherein the yield of the isolated cells is not high. In existing tissue homogenizers for obtaining cells from tissue, it is not possible to use mechanical slicing, so that the tissue cannot be effectively cut off in order to increase the chance of contact with the enzyme. Therefore, the reaction time is long, and the number of the obtained cells is small.

The Taiwanese patent TW201512395 discloses a method for rapidly isolating adipose cells, in which. the fat tissue is placed in an accommodating space and cut by means of middle shaft blades connected with a driving unit. Moreover, the Chinese Patent CN20472692U discloses that a filter mesh is arranged in the accommodating space, through which the decomposed cells suspended in solution can be concentrated and guided to a material outlet located at the bottom. Patents TW201512395 and CN2047269U propose methods for rapidly isolating adipose cells, however, in the drawings thereof it is shown that these devices use blades similar to those applied in juicers for carrying out tissue cutting. The Taiwanese patent TW201512395 discloses that a rotating speed of the cutting knife is 300 to 3000 rpm, so that the fat tissue is shred at a high rotation speed, because otherwise the fat cells cannot be chopped and separated. Due to the high-rotation-speed operation damage of the valuable stem cells significantly increases and the temperature is increased, so that cell separation becomes ineffective. In addition, when the devices provided by the above-mentioned patents are rotated, fat tissue stuck to the blade is rotated along with the blade, so that the cutting operation fails.

In addition, the Chinese patent CN103070718A discloses a cell separator comprising a purification chamber, wherein a kettle-shaped body is arranged inside the chamber. A sharp cutting tool is arranged inside the kettle-shaped body, wherein the sharp cutting tool comprises two blade shafts, two blade sets respectively attached to the blade shafts and a filter cover arranged outside the sharp cutting tool, wherein the filter cover is provided with a filter hole, the bottom of the kettle-shaped body being provided with a discharging part extending downwards, wherein a base container is arranged at the bottom portion of the discharging part. However, the operation speed of the device according to this patent is relatively high, so that there exists a risk for the cells of being damaged the separated cells becoming inactive.

US Patent No. US 8,882,012 B2 discloses a cell separation apparatus, comprising a container and a separating component being arranged in the container, wherein the separating component is provided with a shaft part and a plurality of blade groups, wherein the plurality of blade groups are combined with the shaft part. In addition, the cell separation apparatus is subjected to a stirring condition. The stirring condition consists in that a rotation speed of the cell separation apparatus is 200 rpm. However, since the application field of the method discloses in this document is different from that of the present invention and no screening device is provided for cell isolation, such method cannot be not used in the application field of the present invention.

Therefore, there exists a pressing need for the development of a novel technology capable of solving the defects known from the prior art.

### Summary of the Invention

The present invention aims to provide a device for isolating cells from tissue, which allows to cut the obtained blocky tissue into small pieces in a short period of time, so as to increase the contact area between the tissue and the digestive enzymes, wherein afterwards digestion of the tissues is carried out by means of enzyme digestion, so that the endothelial cells can be separated from the tissue and are suspended in a digestive solution, wherein a filter structure in the device is used for separating the suspended cells isolated from tissue fragments which cannot be digested by the digested enzymes. The invention aims to provide a tissue homogenizer having a shearing function, the working speed thereof not exceeding 90 rpm, wherein the tissue homogenizer utilizes a cutting method in combination with an enzyme reaction for separating cells from tissues or fats rapidly and effectively.

The tissue may be adipose tissue, not being limited thereto. The separated suspended cells comprise single stem cells

Compared with other similar technologies, the method has the following advantage: according to the prior art, the technologies of separating single cells from tissue are based on the separation steps of enzymatic separation and centrifugation, which is relatively time-consuming and has low yield, so that it needs to be subjected to an in-vitro culture for amplification of the cell quantity.

Another purpose of the present invention is to provide an isolation device using specific mixed enzyme formulas for simultaneously carrying out physical cutting of tissue under the assistance of chemical reagents. With the integration of reagents and steps there is no need for carrying out an in-vitro amplification process, so that the result of obtaining a large number of single cells can be rapidly achieved. The device can be utilized for immediate clinical applications.

The present invention further aims to provide an unique technology for single-time implantation of autologous mesenchymal stem cells from fat tissue.

The device disclosed by the present invention has the following characteristics: the device is provided with a stirring-shearing component and two set-cups capable of being matched with the stirring-shearing component, the set-cups being metal structures, wherein an inner set- cup is provided with a filter element, such as a filter hole or a screen mesh, by means of which solid objects having a specific size can be blocked, wherein an outer set-cup does not contain a filter structure and is used for receiving separated liquid and suspended materials passed through the filter element, so that in this step the cells form the tissues can be effectively dispersed in the digestive enzyme solution.

The device for isolating cells from tissue according to the present invention comprises the following: a stirring-shearing component having a rotatable cutting tool and being arranged on a rotor rotating lever driven by a motor and being used for cutting and stirring a tissue during the decomposition of the same in a solution containing digestive enzyme; the device further comprising an inner set filtering cup for accommodating the stirring-shearing component and a digestive enzyme solution, which is provided with a sieve element for filtering the solution containing the desired single cells cut by the stirring-shearing component; wherein the device further comprises an outer set cup for accommodating the inner set filter cup and the filtered solution with the desired single cells.

### Brief description of the drawing

The drawing for illustrating the invention by way of an example shows

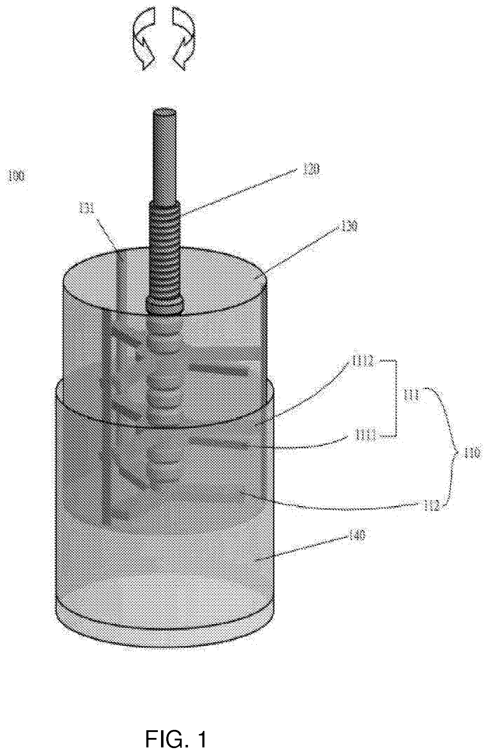

FIG. 1 a schematic diagram of the overall structure of a device for isolating cells from tissue according to the present invention.

### Detailed description of the specific embodiments

The present invention provides a device for rapidly isolating cells from tissue, which substantially comprises a stirring- shearing component for cutting of continuous tissues, wherein the stirring-shearing component is provided with a rotor having spiral blades, wherein an outer set cup is provided with a fixed cutting knife structure, wherein cutting and stirring of tissues are simultaneously thoroughly performed through the rotation of the spiral blades. During operation, a tissue and digestive enzyme solution are simultaneously placed into the device. Through the cutting and stirring and by means of the effects of the digestive enzymes, the block-shaped tissue can be effectively cut into small pieces, so that the contact with the digestive enzymes can be enhanced. The digestive enzymes can digest the tissue, so that the cells can be released from the tissue. The cell tissue suspended in the solution can pass through the filtering member of the inner set cup. After the digestion is completed, the movable spiral cutting blades and the inner set filtering cup are removed. The cell tissue is accommodated in the outer set cup, so that collection of the cells is completed.

Reference is made to FIG. 1. In the present embodiment, a device for isolating cells from tissue 100 according to the present invention comprises the following:
- a stirring and shearing component 110 including a rotatable cutting tool 111 and being arranged on a rotor linkage rod 120, which is driven by a motor (not shown in the figure), and serving for cutting and stirring the tissue during decomposition, when the digestive enzyme solution is added;
- an inner set filtering cup 130 for receiving the stirring and shearing component 110 and a digestive enzyme solution and comprising a sieve component (not shown in the figure) for filtering a solution containing the desired cells cut by the stirring and shearing component 110; and
- an outer set cup 140 for accommodating the inner set filtering cup 130 and the filtered solution with the required single cells.

In the device of the present invention, the rotatable cutting tool 111 is a cutting stirring blade having a plurality of sandwich structures. The cutting stirring blade having a plurality of sandwich structures is respectively provided with a rotating blade 1111 and a static blade 1112, the length of each rotating blade 1111 being smaller than that of the respective static blade 1112, wherein, when the cutting tool 1111 is rotated, each of the static blades 1112 is prevented from rotating by at least one stop member 131 located on the inner wall of the inner set filtering cup 130, so that the static blades 1112 are not movable. The stop member 131 is cylindrical, semicylindrical or elongated strip-shaped

According to another embodiment the rotatable cutting tool 111 comprises a plurality of rotating blades 1111 at a certain vertical distance from each other and a plurality of static blades fixed on the inner wall of the inner set filtering cup 130 (not shown in the figure), wherein each static blade is arranged above or below a corresponding rotating blade 1111, so as to form a cutting stirring knife with a sandwich structure

In accordance with an embodiment of the present invention, the sieve element of the inner set filtering cup 130 comprises a plurality of filtering holes penetrating through the inner set filtering cup (not shown in the figure), wherein the diameter of the plurality of filtering holes is selected to allow the desired cells to pass therethrough and tissue fragments not decomposed by the digestive enzymes solution being blocked by said diameter. In another embodiment, the sieve element of the inner set filtering cup 130 consists of a plurality of filter holes passing through the bottom of the inner set filtering cup and a screen mesh arranged on the cup bottom (not shown in the figure), wherein the mesh size of the screen mesh is smaller than that of the filter holes of the cup bottom, wherein the required cells can pass the mesh, whereas tissue fragments not being decomposed by the digestive enzyme solution are retained by the mesh.

In addition, in the present invention, the stirring and shearing member 110 may be separately removable or can be simultaneously removed with the inner set filtering cup 130, wherein the cutting and stirring blades each comprising multiple sandwich structures are designed in a manner, wherein multiple blades are directly stacked in a plurality of layers.

In the device of the present invention, all static blades (not shown in the figure) respectively arranged on the inner wall of the inner set filtering cup are fixed on the inner wall of the inner set filtering cup by a pin.

In addition, in the device of the invention, the rotor of the motor can be set to rotate in a forward or backward direction, and the rotating speed thereof being less than 90 rpm.

According to the invention, the stirring and shearing member 110 has a base 112 connected thereto that is used for supporting the stirring and shearing component 110.

## Claims

1. A device for isolating cells from tissue, comprising
a stirring and shearing component including a rotatable cutting tool and being arranged on a rotor linkage rod driven by a motor, and configured for cutting and stirring a tissue during decomposition in presence of a digestive enzyme solution;
an inner set filtering cup for receiving the stirring and shearing component and a digestive enzyme solution and being provided with a sieve component configured for filtering the solution cut by the stirring and shearing component and containing the desired isolated cells; and
an outer set cup for receiving inner set filtering cup and the filtered solution with the desired single cells.

2. The device of claim 1, wherein the rotatable cutting tool is a cutting stirring knife having a plurality of sandwich structures, the cutting stirring knife having multiple sandwich structures being provided with a rotating blade and a static blade, wherein the length of each rotating blade is smaller than the length of the respective static blade, wherein at least one stop member is located on the inner wall of the inner set filtering cup for blocking the static blades from rotating, when the cutting tool is rotated, so that the static blades are therefore not movable.

3. The device of claim 1, wherein the rotatable cutting tool comprises a plurality of rotating blades, which are vertically spaced from each other by a certain distance, and a plurality of static blades fixed on the inner wall of the inner set filtering cup, wherein each static blade is correspondingly arranged above or below a corresponding rotating blade, so as to form a cutting stirring knife with a sandwich structure.

4. The device of claim 1, wherein the sieve component of the inner set filtering cup consists of a plurality of filtering holes penetrating through the inner set filtering cup, wherein the diameters of the plurality of filter holes allow the required cells to pass therethrough, whereas tissue fragments not decomposed by the digestive enzyme solution are prevented to pass through the selected diameter.

5. The device of claim 1, wherein the sieve element of the inner set filtering cup consists of a plurality of filtering holes penetrating through the bottom of the inner set filtering cup and a screen mesh arranged at the bottom of the cup, wherein the mesh size of the screen mesh is smaller than that of the filter holes in the cup bottom, wherein the required cells are allowed to pass through the mesh, whereas the mesh retains tissue fragments not being decomposed by the digestive enzyme solution.

6. The device of claim 2, wherein the rotatable cutting tool is provided with a plurality of cutting blades, and the stirring shearing component is independently removable or is simultaneously removable with the inner set filtering cup.

7. The device of claim 2, wherein each of the cutting and stirring blades is formed by directly stacking a plurality of layers by a plurality of blades.

8. The device of claim 2, wherein the stopping component is cylindrical, semicylindrical or elongated strip-shaped.

9. The device of claim 3, wherein the static blades fixed on the inner wall of the inner set filtering cup are respectively mounted on the inner wall of the inner set filtering cup by means of a pin.

10. The device of claim 1, wherein the rotor of the motor is adjustable to perform a forward rotation or reverse rotation, and the rotation speed of the motor being less than 90 rpm.

11. The device of claim 1, wherein the stirring and shearing component has a base connected thereto, which is used for supporting the rotatable cutting tool.
